(19) **Europäisches Patentamt**

**European Patent Office**

**Office européen des brevets**

(11) **EP 0 660 112 B1**

(12) **EUROPÄISCHE PATENTSCHRIFT**

(45) Veröffentlichungstag und Bekanntmachung des Hinweises auf die Patenterteilung:
**05.09.2001 Patentblatt 2001/36**

(51) Int Cl.$^7$: **G01N 33/36**

(21) Anmeldenummer: **94119308.8**

(22) Anmeldetag: **07.12.1994**

(54) **Verfahren zur automatischen Charakterisierung von mechanischen und/oder geometrischen Eigenschaften von Stapelfaserproben sowie dafür geeignete Vorrichtung**

Method for automatically characterising mechanical and/or geometical properties of samples of staple fibers and apparatus suitable therefor

Méthode pour la caractérisation automatique des propriétés mécaniques et/ou géométriques d'échantillons de fibres discontinues et appareil approprié à cet effet

(84) Benannte Vertragsstaaten:
**AT CH DE FR GB LI NL SE**

(30) Priorität: **17.12.1993 DE 4343157**

(43) Veröffentlichungstag der Anmeldung:
**28.06.1995 Patentblatt 1995/26**

(73) Patentinhaber: **TREVIRA GMBH**
**60528 Frankfurt am Main (DE)**

(72) Erfinder:
• **Schneider, August, Dr.**
**D-86845 Grossaitingen (DE)**
• **Bader, Jochen**
**D-86830 Schwabmünchen (DE)**

• **Leimer, Franz Xaver**
**D-86517 Wehringen (DE)**

(74) Vertreter: **Luderschmidt, Schüler & Partner GbR Patentanwälte,**
**Postfach 3929**
**65029 Wiesbaden (DE)**

(56) Entgegenhaltungen:
DE-A- 1 648 802     US-A- 3 063 294
US-A- 4 057 350     US-A- 5 178 007

• **RESEARCH DISCLOSURE, Bd. 124, August 1974, Seite 13 XP002023920 "TOW CRIMP ANALYSER"**

**Beschreibung**

Die vorliegende Erfindung betrifft ein Verfahren zur automatischen Charakterisierung von mechanischen und/oder geometrischen Eigenschaften von Stapelfaserproben, insbesondere von Kräuseleigenschaften, wie die Einkräuselung oder die Anzahl der Kräuselbögen solcher Fasern, sowie eine zur Durchführung dieses Verfahrens geeignete Vorrichtung.

Verfahren zur Charakterisierung von mechanischen und/oder geometrischen Eigenschaften von Fasern, wie zur Bestimmung der Kräuseleigenschaften von Fasern sind an sich bekannt.

Eine der Möglichkeiten, die Kräuseleigenschaften von Fasern zu charakterisieren, beruht auf der Bestimmung der sogenannten "Einkräuselung". Dazu wird die Faser zweimal mit Kräften vorgegebener Größe belastet, wobei die erste Kraft so gering gewählt wird, daß sie noch keine Entkräuselung bewirkt, und die zweite Kraft so groß gewählt wird, daß die Kräuselung vollständig entfernt wird, ohne aber die Faser in Längsrichtung zu dehnen. Aus der Längendifferenz zwischen dem eingekräuselten Zustand und dem gestreckten Zustand der Faser wird eine als "Einkräuselung" bezeichnete Kenngröße abgeleitet. Meßverfahren dieses Typs sind beispielsweise in der DE-A-2,925,810 beschrieben.

Zur Durchführung solcher Messungen hat sich die sogenannte "Kräuselwaage" eingebürgert. Dabei handelt es sich um eine Vorrichtung, in welche eine zu charakterisierende Stapelfaser an beiden Seiten eingeklemmt wird, wobei eine der Klemmen an einem Ende des Waagebalkens angebracht ist. Das andere Ende des Waagebalkens ist mit einer Vorrichtung für die Aufnahme eines ( Massestückes versehen. Dieses Massestück erzeugt die Kraft, die notwendig ist, um die zu prüfende Faser definiert zu entkräuseln.

Die zweite Klemme ist mittels Motorantrieb in der Faserachse beweglich ausgestaltet und sitzt auf der Welle einer Mikrometerschraube. Die Faser wird durch Verfahren der beweglichen Klemme so lange gestreckt, bis die von der Faser aufgenommene Zugkraft jener durch das Massestück am anderen Ende des Waagebalkens erzeugten Kraft entspricht.

Hiermit ist das Gleichgewicht der Waage hergestellt, das photoelektrisch erfaßt und mittels entsprechender Elektronik zum Stop der Klemmenbewegung benutzt wird.

Auf der Mikrometerschraube kann die dem Weg der Klemme entsprechende Ländenänderung ΔL der Faser abgelesen werden.

Es sind bereits optische Messungen zur Bestimmung der Kräuseleigenschaften bekanntgeworden. So wird in der SU-A-1,183,898 eine optische Bestimmung des Kraft-Dehnungsverhaltens von gekräuselten Einzelfasern beschrieben. Aus der US-A-4,057,350 ist ein Verfahren zur Ermittlung der Kräuselung von Faserkabeln bekannt, worin die Streuung eines Laserstrahls als Meßgröße für den Grad der Kräuselung verwendet wird. Weitere optische Meßmethoden zur Bestimmung der Kräuselung von laufenden Faserkabeln sind aus der WO-A-92-2,001, der US-A-4,270,252 und der RD-209,007 bekannt. Die DE-PS-1,473,750 beschreibt die Überwachung der Gleichförmigkeit der Kräuselung von Faserkabeln mittels einer mechanischen Methode.

Aus der EP-B-466,846 ist der Einsatz von Bildverarbeitung bei der Messung von Faserparametern bekannt.

Ferner ist es bekannt, zur Charakterisierung der Kräuselung von Fasern die Anzahl der Kräuselbögen pro Längeneinheit der Faser, bei vorgegebener Faserspannung, zu bestimmen. Zu diesem Zweck wird üblicherweise die Kräuselwaage herangezogen und desweiteren wird die Anzahl der Kräuselbögen der eingespannten Fasern visuell ausgewertet. Das bekannte Verfahren eignet sich nicht zur Automatisierung; außerdem ist es personal- und damit kostenintensiv.

Aus der DE-A-1,648,802 und der US-A-5,178,007 sind Vorrichtungen zum selbsttätigen Zuführen von Proben zu Apparaten zur Charakterisierung von Faserproben bekanntgeworden.

Es wurde jetzt ein Verfahren zur Charakterisierung von mechanischen und/oder geometrischen Eigenschaften von Stapelfasern gefunden, insbesondere zur Charakterisierung der Kräuselung solcher Fasern, das einfach und schnell durchzuführen ist und daß weitgehend automatisiert werden kann.

Die vorliegende Erfindung betrifft ein Verfahren zur automatischen Charakterisierung von mechanischen und/oder geometrischen Eigenschaften von Stapelfaserproben umfassend folgende Maßnahmen:

a) Vorlage von Stapelfaserproben (1) in einem Magazin (3), das mehrere Klemmen (4) zur Befestigung des oberen Endes jeweils einer Stapelfaserprobe (1) enthält, welche Klemmen (4) auf einer Klemmleiste (5) angebracht sind und um eine vorbestimmte Strecke auf oder mit der Klemmleiste (5) beweglich sind,

b) Verbinden des gefüllten Magazins (3) mit einem Apparat (2) zur Charakterisierung von mechanischen und/oder geometrischen Eigenschaften der Stapelfaserproben (1), welcher Apparat mindestens eine obere Klemmbacke (6) und gegebenenfalls eine untere Klemmbacke (7) zur Befestigung der zu charakterisierenden Stapelfaserprobe (1) aufweist, dessen obere Klemmbacke (6) bewegbar ist, wobei das Magazin (3) mit dem Apparat (2) lösbar in einer solchen Weise verbunden ist, so daß die Klemmen (4) des Magazins (3) sich in der Nähe der oberen Klemmbacke (6) des Apparats (2) vorbeibewegen lassen,

c) Bewegen der mit Stapelfaserproben (1) belegten Klemmen (4) des Magazins (3) um eine vorbestimmte Strecke mittels einer Transportvorrichtung (8),

d) Positionieren einer Klemme (4) des Magazins (3) in der Nähe der oberen Klemmbacke (6) des Apparats (2) mittels einer Justiervorrichtung (9),

e) Bewegen der oberen Klemmbacke (6) des Apparats (2) aus einer Meßposition (11) in eine Übernahmeposition (12) mittels einer Transportvorrichtung (10), wobei die obere Klemmbacke (6) in geöffneter Position vorliegt,

f) Schließen der oberen Klemmbacke (6) des Apparats (2) nach Erreichen der Übernahmeposition (12) mittels einer Öffnungs- und Schließvorrichtung (13) unter Übernahme der Stapelfaserprobe (1) aus der Klemme (4) des Magazins (3),

g) Bewegen der oberen Klemmbacke (6) des Apparats (2) zusammen mit der Stapelfaserprobe (1) aus der Übernahmeposition (12) in die Meßposition (11) mittels der Transportvorrichtung (10),

h) Durchführung der Charakterisierung der mechanischen und/oder geometrischen Eigenschaften der Stapelfaserproben (1) mit dem Apparat (2), und

i) Öffnen der oberen Klemmbacke (6) des Apparats (2) nach der Charakterisierung mittels der Öffnungs- und Schließvorrichtung (13) und Entfernung der Stapelfaserprobe (1) aus dem Apparat (2).

Ferner betrifft die Erfindung eine Vorrichtung zur automatischen Charakterisierung von mechanischen und/oder geometrischen Eigenschaften von Stapelfaserproben (1) umfassend folgende Elemente:

A) Magazin (3) zur Aufnahme von Stapelfaserproben (1), das mehrere Klemmen (4) zur Befestigung des oberen Endes jeweils einer Stapelfaserprobe (1) enthält, welche Klemmen (4) auf einer Klemmleiste (5) angebracht sind und welche Klemmen (4) um eine vorbestimmte Länge auf oder mit der Klemmleiste (5) beweglich sind,

B) Apparat (2) zur Charakterisierung von mechanischen und/oder geometrischen Eigenschaften der Stapelfaserproben (1), der mindestens eine obere Klemmbacke (6) und gegebenenfalls eine untere Klemmbacke (7) zur Befestigung der zu charakterisierenden Stapelfaserprobe (1) aufweist, dessen obere Klemmbacke (6) bewegbar ist, wobei das Magazin (3) mit dem Apparat (2) lösbar in einer solchen Weise verbunden ist, so daß die Klemmen (4) des Magazins (3) sich in der Nähe der oberen Klemmbacke (6) des Apparats (2) vorbeibewegen lassen,

C) Transportvorrichtung (8), welche die Klemmen (4) des Magazins (3) um eine vorbestimmte Strecke zu bewegen gestattet,

D) Justiervorrichtung (9), welche die Positionierung einer Klemme (4) des Magazins (3) in der Nähe der oberen Klemmbacke (6) des Apparats (2) gestattet,

E) Transportvorrichtung (10), welche die obere Klemmbacke (6) des Apparats (2) aus einer Meßposition (11) in eine Übernahmeposition (12) und wieder zurück zu bewegen gestattet, und

F) Öffnungs- und Schließvorrichtung (13), welche ein Öffnen und Schließen der oberen Klemmbacke (6) des Apparats (2) gestattet und eine Übernahme der Stapelfaserprobe (1) aus dem Magazin (3) sowie eine Entfernung der Stapelfaserprobe (1) aus dem Apparat (2) gestattet.

Mit dem erfindungsgemäßen Verfahren bzw. der erfindungsgemäßen Vorrichtung lassen sich Stapelfasern aller Art charakterisieren. Dabei kann es sich um ungekräuselte insbesondere jedoch um gekräuselte Stapelfasern handeln.

Mit dem erfindungsgemäßen Verfahren bzw. der erfindungsgemäßen Vorrichtung lassen sich im Prinzip alle Messungen ausführen, die zur Charakterisierung von mechanischen und/oder geometrischen Eigenschaften von Stapelfaserproben herangezogen werden können.

Beispiele für die Charakterisierung von mechanischen Eigenschaften sind die Aufnahme von Kraft-Dehnungs Diagrammen (KD-Diagrammen), die Messung des Schrumpfes, der Schrumpfkraft, der Kräuselung oder eine Kombination dieser Messungen.

In einer besonders bevorzugten Ausführungsform der Erfindung werden die Kräuseleigenschaften von Stapelfasern ermittelt. Die Bestimmung der Kräuseleigenschaften kann beispielsweise folgende Meßvorgänge einzeln oder in Kombination umfassen:

1. Bestimmung der Anzahl der Kräuselbögen pro Längeneinheit der Faser; diese Meßmethode wird weiter unten im einzelnen beschrieben

2. Bestimmung des Einkräuselungskennwertes $K_1$ nach der Beziehung

$$K_1 = (L_0 - L_1) / L_1$$

Dabei bedeutet $L_0$ die definierte Einspannlänge der gekräuselten Faser und $L_1$ ist die Länge der Faser beim Erreichen der Entkräuselungskraft $F_{EK}$.

Die Entkräuselungskraft $F_{EK}$ ist diejenige Kraft, die benötigt wird, um die Faser in einer ersten Belastung zu entkräuseln. $F_{EK}$ kann in einem separaten Meßgang ermittelt werden oder gleichzeitig bei der Bestimmung von $L_1$.

3. Bestimmung des Restkräuselungskennwertes $K_2$ nach der Beziehung

$$K_2 = (L_0 - L_2) / L_2$$

Dabei bedeutet $L_0$ die definierte Einspannlänge der gekräuselten Faser und $L_2$ ist die Länge der vorbelasteten Faser beim Erreichen der Entkräuselungskraft $F_{EK}$. Die Vorbelastung der Faser bei der Ermittlung von $K_2$ kann nach der Ermittlung von $K_1$ erfolgen und beinhaltet folgende Maßnahmen:

3.1. Einwirkung einer vorgegebenen Beständigkeitskraft $F_B$ über eine vorgegebene Dauer auf die Faser, beispielsweise $F_B = 0,5$ cN/dtex

3.2. Entlastung der Faser über die Positionierung der unteren Klemmbacke (7) auf eine Einspannlänge $< L_0$ und Erholungsphase der Faser über eine vorgegebene Dauer durch Verharren der unteren Klemmbacke (7) in dieser Position

4. Bestimmung des Kennwertes der Kräuselbeständigkeit KB durch Bildung des Quotienten aus $K_1$ und $K_2$, beispielsweise nach der Beziehung

$$KB = K_2 / K_1$$

Die Angabe von $K_1$ und $K_2$ kann auch in % erfolgen.

Ein bevorzugtes Beispiel für die Charakterisierung der geometrischen Eigenschaften ist die Ermittlung der Zahl der Kräuselbögen von Stapelfasern.

Unter dem Begriff "Stapelfaserprobe" sind im Rahmen dieser Beschreibung sowohl Bündel von Stapelfasern als auch vorzugsweise einzelne Stapelfasern zu verstehen.

Hinsichtlich des Einzelfasertiters und des faserbildenden Materials ist das erfindungsgemäße Verfahren keinen Beschränkungen unterworfen.

Typische Einzelfasertiter belaufen sich auf 1 bis 20 dtex.

Typische faserbildende Materialien sind Polyphenylensulfid, Polyetherketon, Glas oder carbonsiertes Polyacrylnitril (Kohlenstofffasern), Polyacrylnitril, Polyamide einschließlich der Aramide, und Polyester, insbesondere Polyethylenterephthalat.

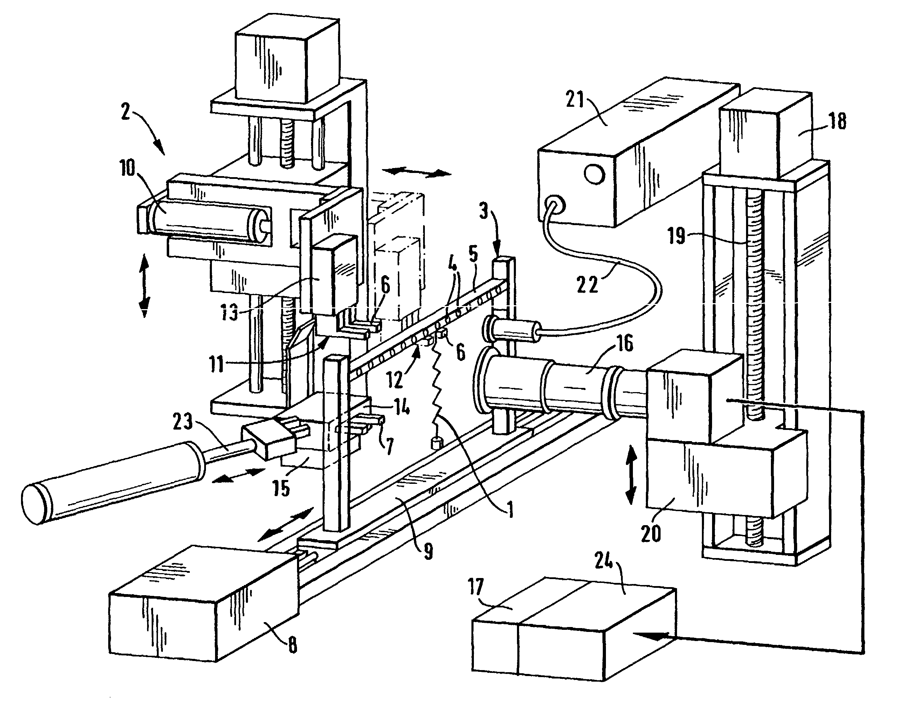

Die Erfindung wird beispielhaft in der Abbildung beschrieben.

Die Vorlage der zu charakterisierenden Stapelfaserproben (1) erfolgt in einem Magazin (3). Dieses Magazin beinhaltet eine Klemmleiste (5), welche die Klemmen (4) trägt. Die Beschickung der Klemmen (4) kann manuell oder automatisch erfolgen.

Dazu werden die oberen Enden der Stapelfaserproben (1) jeweils an Klemmen (4) befestigt. Die unteren Enden der Stapelfaserproben (1) können dabei frei im Magazin (3) hängen oder diese werden vorzugsweise mit einem Massestück vorbestimmter Größe beschwert. Dieses Massestück wird üblicherweise zusammen mit der Stapelfaserprobe (1) in den Apparat (2) mit übernommen.

Die Klemmen (4) sind im Magazin (3) auf der Klemmleiste (5) angebracht und sind gegebenenfalls lösbar mit dieser Klemmleiste (5) verbunden. Die Klemmen (4) sind auf der Klemmleiste (5) beweglich angebracht oder sie können zusammen mit dieser bewegt werden. Bei der Klemmleiste (5) kann es sich um einen horizontal im Magazin (3) angebrachten Stab handeln. Dieser kann Vorrichtungen aufweisen, an denen wiederum die Klemmen (4) lösbar angebracht, beispielsweise festgeklemmt sind. Vorzugsweise verbleiben die Klemmen (4) nach der Übernahme der Stapelfaserprobe (1) in den Apparat (2) im Magazin (3).

Nach dem Befüllen des Magazins (3) mit Stapelfaserproben (1) wird dieses mit dem Apparat (2) zur Charakterisierung von mechanischen und/oder geometrischen Eigenschaften der Stapelfaserproben (1) manuell oder automatisch verbunden. Dies kann über eine beliebige mechanische Verbindung erfolgen, die es gestattet, die Position des Magazins (3) in Bezug auf den Apparat (2) zu fixieren.

Unter dem Begriff "Verbinden des gefüllten Magazins (3) mit dem Apparat (2)" ist auch eine Ausführungsform zu verstehen, bei der lediglich die Klemmleiste (5) zwecks Beschickung mit Stapelfaserproben (1) entnommen und anschließend wieder im Magazin (3) fixiert wird.

Apparat (2) weist mindestens eine obere Klemmbacke (6) zur Befestigung der zu charakterisierenden Stapelfaserprobe (1) auf. Gegebenenfalls ist noch eine untere Klemmbacke (7) vorgesehen. Zur Charakterisierung wird die Stapelfaserprobe (1) in die obere Klemmbacke (6) bzw. in die obere Klemmbacke (6) und in die untere Klemmbacke (7) eingespannt.

Zum Zweck der Übernahme einer Stapelfaserprobe (1) aus dem Magazin (3) wird eine mit einer Stapelfaserprobe (1) belegte Klemme (4) des Magazins (3) um eine vorbestimmte Strecke mittels einer Transportvorrichtung (8) bewegt und mittels einer Justiervorrichtung (9) in der Nähe, vorzugsweise über der oberen Klemmbacke (6) des Apparats (2) positioniert. Anstelle der Klemme (4) kann auch der Apparat (2) bewegt werden.

Anschließend wird die obere Klemmbacke (6) des Apparats (2) aus einer Meßposition (11) in eine Übernahmeposition (12) mittels einer Transportvorrichtung (10) bewegt. Dabei liegt die obere Klemmbacke (6) in geöffneter Position vor.

Die Bewegung der oberen Klemmbacke (6) erfolgt dabei vorzugsweise in horizontaler Richtung und im rechten Winkel zur Bewegungsrichtung der Klemmen (4) des Magazins (3).

Nach dem Erreichen einer Übernahmeposition (12) wird die obere Klemmbacke (6) des Apparats (2) geschlossen und erfaßt die Stapelfaserprobe (1). Dies erfolgt mittels einer Öffnungs- und Schließvorrichtung (13).

Zur Übernahme der Stapelfaserprobe (1) in den Apparat (2) wird die geschlossene obere Klemmbacke (6) zusammen mit der Stapelfaserprobe (1) und gegebenenfalls mit der Klemme (4) aus der Übernahmeposition (12) in die Meßposition (11) bewegt. Dies erfolgt mittels der Transportvorrichtung (10). Die Bewegung der oberen Klemmbacke (6) und der darin eingeklemmten Stapelfaserprobe (1) bewirkt eine Übernahme der Stapelfaserprobe (1) in den Apparat (2).

Nach Erreichen der Meßposition kann die Charakterisierung der mechanischen und/oder geometrischen Eigenschaften der Stapelfaserprobe (1) mit dem Apparat (2) durchgeführt werden.

Je nach gewünschter Meßmethode kann die Stapelfaserprobe (1) direkt nach der Übernahme charakterisiert werden oder es erfolgt vorher noch eine weitere Positionierung bzw. Fixierung der Stapelfaserprobe (1), beispielsweise durch Festklemmen des unteren Teils der Stapelfaserprobe (1) in einer unteren Klemmbacke (7) des Apparats (2) mittels einer Öffnungs- und Schließvorrichtung (14).

Nach der Charakterisierung der Stapelfaserprobe (1) im Apparat (2) wird die obere Klemmbacke (6) und gegebenenfalls die untere Klemmbacke (7) mittels der Öffnungs- und Schließvorrichtungen (13) und (14) geöffnet und die Stapelfaserprobe (1) aus dem Apparat (2) entfernt.

In einer bevorzugten Ausführungsform der Erfindung wird die Einkräuselung der Stapelfaserprobe (1) bestimmt, wobei die erforderliche Messung der Vorspannung mittels eines nicht dargestellten Kraftsensors (15) vorgenommen wird, auf den die obere Klemmbacke (6) oder die untere Klemmbacke (7) einwirkt.

In der bevorzugten Ausführungsform gemäß Abbildung ist die automatische Bestimmung der Anzahl der Kräuselbögen pro Längeneinheit von gekräuselten Stapelfasern dargestellt.

Danach wird die Anzahl der Kräuselbögen pro Längeneinheit der Stapelfaserprobe (1) mit dem Apparat (2) in folgender Weise bestimmt:

h3) Erzeugung einer Abbildung vorgegebener Länge und vorgegebener Breite von der Stapelfaserprobe (1) mittels einer Abbildungsvorrichtung (16), welche vorzugsweise in Richtung der Faserlängsachse bewegbar ist,

h4) Erzeugung eines digitalen Rasters aus der Abbildung, dessen Pixel in Form von Zahlenwerten in einer Speichervorrichtung (17) abgelegt werden, wobei die Zahlenwerte Maßzahlen für die Helligkeit an dem jeweiligen Ort der Abbildung darstellen, und

h5) Ermittlung der Anzahl der Kräuselbögen der abgebildeten Stapelfaserprobe aus dem digitalen Raster mittels digitaler Bildverarbeitung.

In der in der Abbildung dargestellten bevorzugten Ausführungsform weist die Vorrichtung eine Abbildungsvorrichtung (16) auf, die zur Erzeugung einer Abbildung vorgegebener Länge und vorgegebener Breite von der sich im Apparat (2) befindenden Stapelfaserprobe (1) dient und die in Richtung der Faserlängsachse bewegbar ist und welche Abbildungsvorrichtung (16) eine Datenverarbeitungsanlage (24) ansteuert, welche ein digitales Raster aus der Abbildung erzeugt, dessen Pixel in Form von Zahlenwerten in der Speichervorrichtung (17) abgelegt werden. Anstelle einer in Richtung der Faserlängen bewegbaren Abbildungsvorrichtung (16) läßt sich eine Abbildung mittels einer ortsfest angebrachten Abbildungsvorrichtung (16) erzeugen und als digitales Raster, wie oben beschrieben, abspeichern und verarbeiten.

Unter Bildverarbeitung im Sinne dieser Beschreibung ist die Analyse von Bildern bzw. die Rekonstruktion von Objekten aus ihrer Ansicht zu verstehen. Die Bildverarbeitung dient der Mustererkennung. Die Mustererkennung wird zur Merkmalsextraktion und zur Klassifikation herangezogen.

In einer besonders bevorzugten Ausführungsform des erfindungsgemäßen Verfahrens wird die digitale Bildverarbeitung in Schritt h5) wie folgt durchgeführt:

h6) Erzeugung einer Faserlängsachse in der Abbildung, welche dem Verlauf entspricht, den die Stapelfaserprobe (1) in gestrecktem Zustand aufweisen würde,

h7) Erzeugung einer Mittellinie in der Abbildung, welche Mittellinie in der Faserlängsachse nach Schritt h6) oder in vorgegebenem Abstand parallel zu besagter Faserlängsachse verläuft und die in Schritt h3) erzeugte Abbildung der Stapelfaserprobe (1) mindestens mehrfach schneidet, und

h8) Ermitteln der Anzahl der Schnittpunkte zwischen der in Schritt h7) erzeugten Mittellinie und der in Schritt h3) erzeugten Abbildung der Stapelfaserprobe (1) als Maß für die Anzahl der in der Abbildung auftretenden Kräuselbögen der Stapelfaserprobe (1).

Die Faserlängsachse kann z.B. durch eine Ausgleichskurve bzw. durch abschnittsweise definierte Ausgleichskurven definiert werden. Die Daten zur Berechnung der Ausgleichskurve werden über die Abbildungsvorrichtung (16) über die Datenverarbeitungsanlage (24) in der Speichervorrichtung (17) abgelegt. Die Berechnung der Ausgleichskurve erfolgt in der Datenverarbeitungsanlage (24).

Die Mittellinie in Schritt h7) kann in der Faserlängsachse nach Schritt h6) verlaufen oder durch einen Offset-Wert auf einen der Parameter der Ausgleichskurve erzeugt werden.

Die Ermittlung der Anzahl der Schnittpunkte in Schritt h8) kann durch Ermittlung des Nulldurchgangs der Differenzkurve aus Mittellinie und Abbildung der Stapelfaserprobe (Kräuselkurve) (von Punkt zu Punkt Verbindung der digitalisierten Faserkoordinaten) ermittelt werden.

Bei der Abbildungsvorrichtung (16) kann es sich um eine beliebige Kamera handeln, die eine Abbildung in elektronische Signale zu verwandeln vermag, beispielsweise eine Videokamera.

Bevorzugt ist eine Zeilenkamera, da mit dieser eine genügende Auflösung des zu untersuchenden Abbildes der Faser gegeben ist und auch kleine und kleinste herstellungsbedingte Kräuselbögen differenziert werden können.

In einer besonders bevorzugten Ausführungsform werden mit der Zeilenkamera jeweils quer zur Stapelfaserprobe (1) 2048 Pixel abgebildet. Damit ist eine Auflösung von 0,01 mm möglich, was eine Abbildung aller üblicherweise auftretenden Kräuselbögen zuläßt.

Die Bewegung der Abbildungsvorrichtung (16) in Richtung der Faserlängsachse erfolgt bevorzugt mittels eines Schrittmotors (18).

In der in der Abbildung dargestellten bevorzugten Variante wirkt der Schrittmotor (18) über eine Spindel (19) auf eine am Apparat (2) angebrachte Befestigungsvorrichtung (20) ein, an der die Abildungsvorrichtung (16) angebracht ist.

In der in der Abbildung dargestellten bevorzugten Variante wird die Abbildungsvorrichtung (16) bei der Erzeugung der Abbildung von einer Lichtquelle (21) in einer solchen Weise ausgeleuchtet, so daß sich die Stapelfaserprobe (1) im Schnittpunkt zwischen der Lichtquelle (21) und der Abbildungsvorrichtung (16) befindet.

Besonders bevorzugt wird als Lichtquelle (21) ein nicht dargestellter Spiegel eingesetzt, der von einem Lichtwellenleiter (22) bestrahlt wird und die Abbildungsvorrichtung (16) indirekt ausleuchtet. Mit dieser Ausführungsform ist eine besonders geringe thermische Belastung der Stapelfaserprobe (1) verbunden.

Besonders bevorzugt für die Beleuchtung wird eine Kaltlichtlampe, die über einen Spiegel die Abildungsvorrichtung (16) indirekt ausleuchtet. Mit dieser Ausführungsform ist eine besonders günstige thermische Belastung der Stapelfaserprobe (1) verbunden.

Das Magazin (3) kann die Stapelfaserproben (1) beliebiger Anordnung enthalten, vorzugsweise in linearer Anordnung oder in kreisförmiger Anordnung.

In der in der Abbildung dargestellten bevorzugten Variante wird die Stapelfaserprobe (1) nach der Charakterisierung der mechanischen und/oder geometrischen Eigenschaften durch einen Arm (23) aus dem Apparat (2) entfernt. Die Verwendung eines pneumatisch oder hydraulisch betriebenen Arms (23) ist besonders bevorzugt, da bei der Entfernung der Stapelfaserprobe kein Luftzug erwünscht ist.

## Patentansprüche

**1.** Verfahren zur automatischen Charakterisierung von mechanischen und/oder geometrischen Eigenschaften von Stapelfaserproben umfassend folgende Maßnahmen:

    a) Vorlage von Stapelfaserproben (1) in einem Magazin (3), das mehrere Klemmen (4) zur Befestigung des oberen Endes jeweils einer Stapelfaserprobe (1) enthält, welche Klemmen (4) auf einer Klemmleiste (5) angebracht sind und um eine vorbestimmte Strecke auf oder mit der Klemmleiste (5) beweglich sind,

    b) Verbinden des gefüllten Magazins (3) mit einem Apparat (2) zur Charakterisierung von mechanischen und/oder geometrischen Eigenschaften der Stapelfaserproben (1), welcher Apparat mindestens eine obere Klemmbacke (6) und gegebenenfalls eine untere Klemmbacke (7) zur Befestigung der zu charakterisierenden Stapelfaserprobe (1) aufweist, dessen obere Klemmbacke (6) bewegbar ist, wobei das Magazin (3) mit dem Apparat (2) lösbar in einer solchen Weise verbunden ist, so daß die Klemmen (4) des Magazins (3) sich in der Nähe der oberen Klemmbacke (6) des Apparats (2) vorbeibewegen lassen,

    c) Bewegen der mit Stapelfaserproben (1) belegten Klemmen (4) des Magazins (3) um eine vorbestimmte Strecke mittels einer Transportvorrichtung (8),

    d) Positionieren einer Klemme (4) des Magazins (3) in der Nähe der oberen Klemmbacke (6) des Apparats (2) mittels einer Justiervorrichtung (9),

    e) Bewegen der oberen Klemmbacke (6) des Apparats (2) aus einer Meßposition (11) in eine Übernahmeposition (12) mittels einer Transportvorrichtung (10), wobei die obere Klemmbacke (6) in geöffneter Position vorliegt,

    f) Schließen der oberen Klemmbacke (6) des Apparats (2) nach Erreichen der Übernahmeposition (12) mittels einer Öffnungs- und Schließvorrichtung (13) unter Übernahme der Stapelfaserprobe (1) aus der Klemme (4) des Magazins (3),

g) Bewegen der oberen Klemmbacke (6) des Apparats (2) zusammen mit der Stapelfaserprobe (1) aus der Übernahmeposition (12) in die Meßposition (11) mittels der Transportvorrichtung (10),

h) Durchführung der Charakterisierung der mechanischen und/oder geometrischen Eigenschaften der Stapelfaserproben (1) mit dem Apparat (2), und

i) Öffnen der oberen Klemmbacke (6) des Apparats (2) nach der Charakterisierung mittels der Öffnungs- und Schließvorrichtung (13) und Entfernung der Stapelfaserprobe (1) aus dem Apparat (2).

2.  Verfahren nach Anspruch 1, **dadurch gekennzeichnet**, daß bei der Vorlage von Stapelfaserproben (1) in Schritt a) diese jeweils an ihrem unteren Ende mit einem Massestück vorbestimmter Größe beschwert sind.

3.  Verfahren zur automatischen Bestimmung der Einkräuselung von gekräuselten Stapelfasern nach Anspruch 1 umfassend folgende Maßnahmen:

    a1) Vorlage von gekräuselten Stapelfaserproben (1) in Schritt a),

    d1) Vorgabe von oberer Klemmbacke (6) und unterer Klemmbacke (7) in geöffneter Position in Schritt d),

    g1) Schließen der unteren Klemmbacke (7) des Apparats (2) nach der Bewegung der oberen Klemmbacke (6) in die Meßposition (11) (Schritt g)) mittels einer Öffnungs- und Schließvorrichtung (14) unter Einspannen des unteren Endes der Stapelfaserprobe (1) mit der unteren Klemmbacke (7),

    h1) Bestimmung der Einkräuselung der Stapelfaserprobe (1) mit dem Apparat (2) in an sich bekannter Weise in Schritt h), und

    i1) Öffnen der oberen und der unteren Klemmbacke (6, 7) des Apparats (2) nach der Charakterisierung mittels den Öffnungs- und Schließvorrichtungen (13, 14) und Entfernung der Stapelfaserprobe (1) aus dem Apparat (2) in Schritt i).

4.  Verfahren nach Anspruch 3, **dadurch gekennzeichnet**, daß der Einkräuselungskennwert $K_1$ nach der Beziehung

$$K_1 = (L_0 - L_1)/L_1$$

ermittelt wird, wobei $L_0$ die definierte Einspannlänge der gekräuselten Faser und $L_1$ die Länge der Faser beim Erreichen der Entkräuselungskraft $F_{EK}$ bedeuten, und $F_{EK}$ diejenige Kraft ist, die benötigt wird, um die Faser in einer ersten Belastung zu entkräuseln.

5.  Verfahren nach Anspruch 3, **dadurch gekennzeichnet**, daß der Restkräuselungskennwert $K_2$ nach der Beziehung

$$K_2 = (L_0 - L_2) / L_2$$

ermittelt wird, wobei $L_0$ die definierte Einspannlänge der gekräuselten Faser und $L_2$ die Länge der vorbelasteten Faser beim Erreichen der Entkräuselungskraft $F_{EK}$ bedeuten, und die Vorbelastung der Faser bei der Ermittlung von $K_2$ folgende Maßnahmen beinhaltet:

    -   Einwirkung einer vorgegebenen Beständigkeitskraft $F_B$ über eine vorgegebene Dauer auf die Faser, und

    -   Entlastung der Faser über die Positionierung der unteren Klemmbacke (7) auf eine Einspannlänge $< L_0$ und Erholungsphase der Faser über eine vorgegebene Dauer durch Verharren der unteren Klemmbacke (7) in dieser Position.

6.  Verfahren nach Anspruch 3, **dadurch gekennzeichnet**, daß der Kennwert der Kräuselbeständigkeit KB nach der Beziehung

$$KB = K_2 / K_1$$

erzeugt wird, wobei $K_1$ und $K_2$ die in den Ansprüchen 4 und 5 angegebenen Definitionen besitzen.

7.  Verfahren nach Anspruch 3, **dadurch gekennzeichnet**, daß die bei der Bestimmung der Einkräuselung der Stapelfaserprobe (1) erforderliche Messung der Faserspannung Faserspannung mittels eines Kraftsensors (15) vorgenommen wird, auf den die obere Klemmbacke (6) oder die untere Klemmbacke (7) einwirkt.

8.  Verfahren zur automatischen Bestimmung der Anzahl der Kräuselbögen pro Längeneinheit von gekräuselten Stapelfasern nach Anspruch 1 umfassend folgende Maßnahmen:

a1) Vorlage von gekräuselten Stapelfaserproben (1) in Schritt a), und

h2) Bestimmung der Anzahl der Kräuselbögen pro Längeneinheit der Stapelfaserprobe (1) mit dem Apparat (2) in folgender Weise in Schritt h)

h3) Erzeugung einer Abbildung vorgegebener Länge und vorgegebener Breite von der Stapelfaserprobe (1) mittels einer Abbildungsvorrichtung (16), welche vorzugsweise in Richtung der Faserlängsachse bewegbar ist

h4) Erzeugung eines digitalen Rasters aus der Abbildung, dessen Pixel in Form von Zahlenwerten in einer Speichervorrichtung (17) abgelegt werden, wobei die Zahlenwerte Maßzahlen für die Helligkeit an dem jeweiligen Ort der Abbildung darstellen, und

h5) Ermittlung der Anzahl der Kräuselbögen der abgebildeten Stapelfaserprobe aus dem digitalen Raster mittels digitaler Bildverarbeitung.

9. Verfahren nach Anspruch 8, **dadurch gekennzeichnet**, daß bei der Vorlage von Stapelfaserproben (1) in Schritt a) diese jeweils an ihrem unteren Ende mit einem Massestück vorbestimmter Größe beschwert sind und daß die so beschwerten Stapelfaserproben (1) in den Apparat (2) übernommen werden.

10. Verfahren zur automatischen Bestimmung der Anzahl der Kräuselbögen pro Längeneinheit von gekräuselten Stapelfasern nach Anspruch 8 umfassend folgende Maßnahmen:

d1) Vorgabe von oberer Klemmbacke (6) und unterer Klemmbacke (7) in geöffneter Position in Schritt d),

g1) Schließen der unteren Klemmbacke (7) des Apparats (2) nach der Bewegung der oberen Klemmbacke (6) in die Meßposition (11) (Schritt g)) mittels einer Öffnungs- und Schließvorrichtung (14) unter Einspannen des unteren Endes der Stapelfaserprobe (1) mit der unteren Klemmbacke (7), und

i1) Öffnen der oberen und der unteren Klemmbacke (6, 7) des Apparats (2) nach der Charakterisierung mittels den Öffnungs- und Schließvorrichtungen (13, 14) und Entfernung der Stapelfaserprobe (1) mit Klemme (4) aus dem Apparat (2) in Schritt i).

11. Verfahren nach Anspruch 8, **dadurch gekenn-**

**zeichnet**, daß neben der Bestimmung der Anzahl der Kräuselbögen pro Längeneinheit der Stapelfaserprobe (1) auch die Bestimmung der Faserspannung, unter welcher sich die Stapelfaserprobe (1) bei der Erzeugung der Abbildung befindet, erfolgt, wobei diese mittels eines Kraftsensors (15) vorgenommen wird, auf den die obere Klemmbacke (6) oder die untere Klemmbacke (7) einwirkt.

12. Verfahren nach Anspruch 8, **dadurch gekennzeichnet**, daß die digitale Bildverarbeitung in Schritt h5) wie folgt durchgeführt wird:

h6) Erzeugung einer Faserlängsachse in der Abbildung, welche dem Verlauf entspricht, den die Stapelfaserprobe (1) in gestrecktem Zustand aufweisen würde,

h7) Erzeugung einer Mittellinie in der Abbildung, welche Mittellinie in der Faserlängsachse nach Schritt h6) oder in vorgegebenem Abstand parallel zu besagter Faserlängsachse verläuft und die in Schritt h3) erzeugte Abbildung der Stapelfaserprobe (1) mindestens mehrfach schneidet, und

h8) Ermitteln der Anzahl der Schnittpunkte zwischen der in Schritt h7) erzeugten Mittellinie und der in Schritt h3) erzeugten Abbildung der Stapelfaserprobe (1) als Maß für die Anzahl der in der Abbildung auftretenden Kräuselbögen der Stapelfaserprobe (1).

13. Verfahren nach Anspruch 8, **dadurch gekennzeichnet**, daß es sich bei der Abbildungsvorrichtung (16) um eine Zeilenkamera handelt.

14. Verfahren nach Anspruch 8, **dadurch gekennzeichnet**, daß die Bewegung der Abbildungsvorrichtung (16) in Richtung der Faserlängsachse mittels eines Schrittmotors (18) erfolgt.

15. Verfahren nach Anspruch 14, **dadurch gekennzeichnet**, daß der Schrittmotor (18) über eine Spindel (19) auf eine am Apparat (2) angebrachten Befestigungsvorrichtung (20) einwirkt, an der sich die Abildungsvorrichtung (16) angebracht ist.

16. Verfahren nach Anspruch 8, **dadurch gekennzeichnet**, daß bei der Erzeugung der Abbildung die Abbildungsvorrichtung (16) von einer Lichtquelle (21) in der Weise ausgeleuchtet wird, so daß sich die Stapelfaserprobe (1) zwischen der Lichtquelle (21) und der Abbildungsvorrichtung (16) befindet.

17. Verfahren nach Anspruch 16, **dadurch gekennzeichnet**, daß als Lichtquelle (21) ein Spiegel eingesetzt wird, der von einem Lichtwellenleiter (22) bestrahlt wird und die Abbildungsvorrichtung (16) indirekt ausleuchtet.

18. Verfahren nach Anspruch 8, **dadurch gekennzeichnet**, daß für die Beleuchtung eine Kaltlichtlampe eingesetzt wird, die über einen Spiegel die Abbildungsvorrichtung (16) indirekt ausleuchtet.

19. Verfahren nach Anspruch 1, **dadurch gekennzeichnet**, daß das Magazin (3) die Stapelfaserproben (1) in linearer Anordnung enthält.

20. Verfahren nach Anspruch 1, **dadurch gekennzeichnet**, daß das Magazin (3) die Stapelfaserproben (1) in kreisförmiger Anordnung enthält.

21. Verfahren nach Anspruch 1, **dadurch gekennzeichnet**, daß die Stapelfaserprobe (1) nach der Charakterisierung der mechanischen und/oder geometrischen Eigenschaften durch einen pneumatisch oder hydraulisch betriebenen Arm (23) aus dem Apparat (2) entfernt wird.

22. Vorrichtung zur automatischen Charakterisierung von mechanischen und/oder geometrischen Eigenschaften von Stapelfaserproben (1) umfassend folgende Elemente:

    A) Magazin (3) zur Aufnahme von Stapelfaserproben (1), das mehrere Klemmen (4) zur Befestigung des oberen Endes jeweils einer Stapelfaserprobe (1) enthält, welche Klemmen (4) auf einer Klemmleiste (5) angebracht sind und welche Klemmen (4) um eine vorbestimmte Länge auf oder mit der Klemmleiste (5) beweglich sind,

    B) Apparat (2) zur Charakterisierung von mechanischen und/oder geometrischen Eigenschaften der Stapelfaserproben (1), der mindestens eine obere Klemmbacke (6) und gegebenenfalls eine untere Klemmbacke (7) zur Befestigung der zu charakterisierenden Stapelfaserprobe (1) aufweist, dessen obere Klemmbacke (6) bewegbar ist, wobei das Magazin (3) mit dem Apparat (2) lösbar in einer solchen Weise verbunden ist, so daß die Klemmen (4) des Magazins (3) sich in der Nähe der oberen Klemmbacke (6) des Apparats (2) vorbeibewegen lassen,

    C) Transportvorrichtung (8), welche die Klemmen (4) des Magazins (3) um eine vorbestimmte Strecke zu bewegen gestattet,

    D) Justiervorrichtung (9), welche die Positionierung einer Klemme (4) des Magazins (3) in der Nähe der oberen Klemmbacke (6) des Apparats (2) gestattet,

    E) Transportvorrichtung (10), welche die obere Klemmbacke (6) des Apparats (2) aus einer Meßposition (11) in eine Übernahmeposition (12) und wieder zurück zu bewegen gestattet, und

    F) Öffnungs- und Schließvorrichtung (13), welche ein Öffnen und Schließen der oberen Klemmbacke (6) des Apparats (2) gestattet und eine Übernahme der Stapelfaserprobe (1) aus dem Magazin (3) sowie eine Entfernung der Stapelfaserprobe (1) aus dem Apparat (2) gestattet.

23. Vorrichtung nach Anspruch 22, **dadurch gekennzeichnet**, daß Apparat (2) zur Bestimmung der Faserspannung einen Kraftsensor (15) aufweist, auf den die obere Klemmbacke (6) oder die untere Klemmbacke (7) einwirkt.

24. Vorrichtung nach Anspruch 22, **dadurch gekennzeichnet**, daß diese eine Abbildungsvorrichtung (16) aufweist, die zur Erzeugung einer Abbildung vorgegebener Länge und vorgegebener Breite von der sich im Apparat (2) befindenden Stapelfaserprobe (1) dient und die vorzugsweise in Richtung der Faserlängsachse bewegbar ist und welche Abbildungsvorrichtung (16) eine Datenverarbeitungsanlage (24) ansteuert, welche ein digitales Raster aus der Abbildung erzeugt, dessen Pixel in Form von Zahlenwerten in einer Speichervorrichtung (17) abgelegt werden, wobei die Zahlenwerte Maßzahlen für die Helligkeit an dem jeweiligen Ort der Abbildung darstellen.

25. Vorrichtung nach Anspruch 24, **dadurch gekennzeichnet**, daß die Datenverarbeitungsanlage (24) die Ermittlung der Anzahl der Kräuselbögen der abgebildeten Stapelfaserprobe aus dem digitalen Raster mittels digitaler Bildverarbeitung gestattet.

26. Vorrichtung nach Anspruch 24, **dadurch gekennzeichnet**, daß es sich bei der Abbildungsvorrichtung (16) um eine Zeilenkamera handelt.

27. Vorrichtung nach Anspruch 24, **dadurch gekennzeichnet**, daß diese einen Schrittmotor (18) aufweist, der die Bewegung der Abbildungsvorrichtung (16) in Richtung der Faserlängsachse bewirkt.

28. Vorrichtung nach Anspruch 27, **dadurch gekennzeichnet**, daß diese eine Spindel (19) aufweist, über welche der Schrittmotor (18) auf eine am Apparat (2) angebrachte Befestigungsvorrichtung (20) einwirkt, an der die Abildungsvorrichtung (16) angebracht ist.

**29.** Vorrichtung nach Anspruch 24, **dadurch gekennzeichnet**, daß diese eine Lichtquelle (21) aufweist, welche bei der Erzeugung der Abbildung die Abbildungsvorrichtung (16) in der Weise ausleuchtet, so daß sich die Stapelfaserprobe (1) im Schnittpunkt zwischen der Lichtquelle (21) und der Abbildungsvorrichtung (16) befindet.

**30.** Vorrichtung nach Anspruch 29, **dadurch gekennzeichnet**, daß die Lichtquelle (21) ein Spiegel ist, der von einem Lichtwellenleiter (22) bestrahlt wird und die Abbildungsvorrichtung (16) indirekt ausleuchtet.

**31.** Vorrichtung nach Anspruch 24, **dadurch gekennzeichnet**, daß die Beleuchtung durch eine Kaltlichtlampe erfolgt, die über einen Spiegel die Abbildungsvorrichtung (16) indirekt ausleuchtet,

**32.** Vorrichtung nach Anspruch 22, **dadurch gekennzeichnet**, daß das Magazin (3) die Stapelfaserproben (1) in linearer Anordnung enthält.

**33.** Vorrichtung nach Anspruch 22, **dadurch gekennzeichnet**, daß das Magazin (3) die Stapelfaserproben (1) in kreisförmiger Anordnung enthält.

**34.** Vorrichtung nach Anspruch 22, **dadurch gekennzeichnet**, daß ein pneumatisch oder hydraulisch betriebener Arm (23) vorgesehen ist, der die Stapelfaserprobe (1) nach der Charakterisierung der mechanischen und/oder geometrischen Eigenschaften aus dem Apparat (2) entfernt.

**Claims**

**1.** A method of automatically characterising mechanical and/or geometrical properties of staple fibre samples and comprising the following measures:

a) presentation of staple fibre samples (10) in a magazine (3) containing a plurality of clamps (4) for fixing the upper end of in each case one staple fibre sample (1), the said clamps (4) being mounted on a clamp strip (5) and being adapted for movement about a predetermined path onto or with the clamp strip (5),

b) connecting the filled magazine (3) with an apparatus (2) for characterising the mechanical and/or geometrical properties of staple fibre samples (1), the said apparatus having at least one upper clamping jaw (6) and, possibly a lower clamping jaw (7) for fixing the staple fibre samples (1) which are to be characterised and of which the upper clamping jaw (6) is movable, whereby the store (3) is, together with the apparatus (2), separably connected in such a way that the clamps (4) of the magazine (3) are able to move past in the vicinity of the upper clamping jaw (6) of the apparatus (2),

c) movement of the clamps (4) of the magazine (3) carrying staple fibre samples (1) through a predetermined path by means of a transport device (8),

d) positioning of a clamp (4) of the magazine (3) in the vicinity of the upper clamping jaw (6) of the apparatus (2) by means of an adjusting device (9),

e) movement of the upper clamping jaw (6) of the apparatus (2) out of a measuring position (11) into a take-over position (12) by means of a transport device (10), the upper clamping jaw (6) being present in the open position,

f) closure of the upper clamping jaw (6) of the apparatus (2) upon reaching the take-over position (12) by means of an opening and closing device (13) and while taking over the staple fibre sample (1) from the clamp (4) of the magazine (3),

g) moving the upper clamping jaw (6) of the apparatus (2) together with the staple fibre sample (1) out of the takeover position (12) into the measuring position (11) by means of the transport device (10),

h) characterising the mechanical and/or geometrical properties of the staple fibre samples (1) with the apparatus (2), and

i) opening the upper clamping jaw (6) of the apparatus (2) after characterising by means of the opening and closing device (13) and removal of the staple fibre sample (1) from the apparatus (2).

**2.** A method according to claim 1, **characterised in that** upon presentation of staple fibre samples (I) in the step a) these are loaded at their bottom end with a piece of predetermined size.

**3.** A method of automatically determining the curl of curled staple fibres according to claim 1 comprising the following measures:

a1) presentation of curled staple fibre samples (1) in step a),

d1) presentation of upper clamping jaw (6) and lower clamping jaw (7) in the open position in step d(,

g1) closure of the lower clamping jaw (7) of the apparatus (2) after movement of the clamping jaw (6) into the measuring position (11) (step g1) by means of an opening and closing device (14), while clamping the bottom end of the staple fibre sample (1) in the lower clamping jaw (7),

h1) determination of the curl of the staple fibre sample (1) with the apparatus (2) in per se known manner in step h), and

i1) opening of the upper and lower clamping jaw (6, 7) of the apparatus (2) after characterisation by means of the opening and closing devices (13, 14) and removal of the staple fibre sample (1) from the apparatus (2) in step i).

4. A method according to claim 3, **characterised in that** the curling characteristic value $K_1$ is ascertained by the equation

$$K_1 = (L_o - L_1) / L_1$$

in which $L_o$ is the defined clamping length of the curled fibre and $L_1$ is the length of the fibre when reaching the decurling force $F_{EK}$ and $F_{EK}$ is that force required in order to uncurl the fibre in a first loading.

5. A method according to claim 3, **characterised in that** the residual curl characteristic $K_2$ is ascertained by the relationship

$$K_2 = (L_o - L_2) / L_2$$

whereby $L_o$ is the defined clamping length of the curled fibre and $L_2$ is the length of the pre-loaded fibre upon attaining the decurling force $F_{EK}$ and the pre-loading of the fibres during measures following ascertainment of $K_2$:

- action of a predetermined resistance force $F_8$ over a predetermined period on the fibre, and

- relieving of the fibres via the position of the lower clamping jaw (7) on a clamping length $<L_o$ and recovering phase of the fibres over a predetermined duration by the bottom clamping jaw (7) remaining fast in this position.

6. A method according to claim 3, **characterised in that** the characteristic value of the curl resistance KB is generated by the relationship

$$KB = K_2 / K_1$$

whereby $K_1$ and $K_2$ have definitions indicated in claims 4 and 5.

7. A method according to claim 3, **characterised in that** the measurement of the fibre tension necessary when determining the curl of the staple fibre sample (1) is undertaken by a force sensor (15) which is acted upon by the upper clamping jaw (6) or lower clamping jaw (7).

8. A method of automatically determining the number of curls per unit of length of curled staple fibres according to claim 1, comprising the following measures:

a1) presentation of curled staple fibre samples (1) in step a), and

h2) determination of the number of curls per unit of length of the staple fibre sample (1) with the apparatus (2) in the following manner in step h),

h3) creation of an illustration of predetermined length and predetermined width by the staple fibre sample (1) by means of a depicting means (16) which can preferably be moved in the direction of the longitudinal axis of the fibre,

h4) generation of a digital screen from the illustration the pixels of which are ascertained in the form of numerical values in a memory device (17), the numerical values constituting numerical measures of the brightness at the particular location of the illustration, and

h5) ascertainment of the number of curls of depicted staple fibre sample from the digital screen by means of digital image processing.

9. A method according to claim 8, **characterised in that** upon presentation of staple fibre samples (1) in step a) this is at the bottom weighted by a mass of predetermined size and in that the staple fibre samples (1) which are so loaded are taken into the apparatus (2).

10. A method of automatically determining the number of curls per unit of length per curled staple fibres according to claim 8 and comprising the following measures:

d1) presentation of upper clamping jaw (6) and lower clamping jaw (7) in the opened position in step d),

g1) closure of the lower clamping jaw (7) of the apparatus (2) after movement of the upper clamping jaw (6) into the measuring position (11) (step g)) by means of an opening and losing device (14) and clamping the bottom end of the staple fibre sample (1) by the lower clamping jaw (7), and

i1) opening of the upper and lower clamping jaws (6, 7) of the apparatus (2) after characterisation by means of the opening and closing devices (13, 14) and removal of the staple fibre sample (1) with clamp (4) from the apparatus (2) in step i).

11. A method according to claim 8, **characterised in that** in addition to determining the number of curls per unit of length of the staple fibre sample (1) there is also determination of the fibre tension under which the staple fibre sample (1) is present when the illustration is made, whereby this is carried out by means of a force sensor (15) which is acted upon by the upper clamping jaw (6) or the lower clamping jaw (7).

12. A method according to claim 8, **characterised in that** the digital image processing in step h5) is carried out as follows:

h6) generation of a longitudinal axis of the fibre in the illustration corresponding to the pattern which the staple fibre sample (1) would follow in the extended state,

h7) generation of a central line in the illustration which central line extends in the longitudinal axis of the fibre after step h6) or at a predetermined distance from and parallel with the said longitudinal axis of the fibre and intersects at least several times the depiction of the staple fibre sample (1) generated in step h3), and

h8) ascertainment of the number of points of intersection between the central line generated in step h7) and the depiction of the staple fibre sample (1) generated in step h3) as a measure of the number of curls in the staple fibre sample (1) which occur in the illustration.

13. A method according to claim 8, **characterised in that** the depiction device (16) is a line-by-line camera.

14. A method according to claim 8, **characterised in that** movement of the depicting device (16) in the direction of the longitudinal axis of the fibre takes place by means of a stepping motor (18).

15. A method according to claim 14, **characterised in that** the stepping motor (18) acts via a spindle (19) on a fixing device (20) mounted on the appliance (2) and on which the depiction device (16) is mounted.

16. A method according to claim 8, **characterised in that** where generation of the depiction of the depicting device (16) is concerned, this is illuminated by a light source (21) in such a manner that the staple fibre sample (1) is disposed between the light source (21) and the depicting device (16).

17. A method according to claim 16, **characterised in that** the light source (21) used is a mirror upon which there is radiation from a light wave conductor (22) while the depicting device (16) is indirectly illuminated.

18. A method according to claim 8, **characterised in that** for lighting, a cold light lamp is used which, via a mirror, indirectly illuminates the deflecting device (16).

19. A method according to claim 1, **characterised in that** the magazine (3) contains the staple fibre samples (I) in a linear arrangement.

20. A method according to claim 1, **characterised in that** the magazine (3) contains the staple fibre samples (I) in a circular arrangement.

21. A method according to claim 1, **characterised in that** the staple fibre sample (1) is, after characterising the mechanical and/or geometrical properties, removed from the apparatus (2) by a pneumatically or hydraulically operated arm (23).

22. A device for automatically characterising mechanical and/or geometrical properties of staple fibre samples (1) and comprising the following elements:

A) a magazine (3) for holding staple fibre samples (1) and which contains a plurality of clamps (4) for fixing the upper end of in each case one staple fibre sample (1), the said clamp (4) being mounted on a clamp strip (5), the said clamps (4) being adapted for movement about a predetermined length or together with the clamping strip (5),

B) an apparatus (2) for characterising mechanical and/or geometrical properties of the staple fibre samples (1) and which comprises at least one upper clamping jaw (6) and possibly one lower clamping jaw (7) for fixing the staple fibre samples (1) which are to be characterised and of which the upper clamping jaw (6) is movable, the magazine (3) being separably connected to the apparatus (2) in such a way that the clamps (4) of the magazine (3) can be moved past and in the vicinity of the upper clamping jaw (6) of the apparatus (2),

C) a transport device (8) which allows the clamps (4) of the magazine (3) to move through a predetermined path,

D) an adjustment device (9) which allows positioning of the clamp (4) of the magazine (3) in the vicinity of the upper jaw (6) of the apparatus (2),

E) a transport device (10) which makes it possible for the upper clamping jaw (6) of the apparatus (2) to be moved out of a measuring position (11) into a takeover position (12) and moved back, and

F) opening and closing device (13) which allows an opening and closing of the upper clamping jaw (6) of the apparatus (2) and a takeover of the staple fibre sample (1) out of the magazine (3) and removal of the staple fibre sample (1) from the apparatus (2).

23. A device according to claim 22, **characterised in that** the apparatus (2) has, for determining the fibre tension, a force sensor (15) which is acted upon by the upper clamping jaw (6) or lower clamping jaw (7).

24. A device according to claim 22, **characterised in that** it comprises a depicting device (16) which serves to create an illustration of predetermined length and predetermined width of the staple fibre sample (1) disposed in the apparatus (2) and which is movable preferably in the direction of the longitudinal axis of the fibre, the said depicting device (16) operating a data processing unit (24) which generates a digital screen from the illustration, the pixels of which are deposited in the form of numerical values in a storage means (17), the numerical values constituting measures of the brightness at the respective location on the illustration.

25. A device according to claim 24, **characterised in that** the data processing unit (24) makes it possible to ascertain the number of curls in the illustrated staple fibre sample from the digital screen, by means of digital image processing.

26. A device according to claim 24, **characterised in that** the depicting device (16) is a line-by-line camera.

27. A device according to claim 24, **characterised in that** this comprises a stepping motor (18) which brings about movement of the depicting device (16) in a direction of the longitudinal axis of the fibre.

28. A device according to claim 27, **characterised in that** it has a spindle (19) via which the stepping motor (18) acts on, provided on the apparatus (2), a fixing device (20) on which the depicting device (16) is mounted.

29. A device according to claim 24, **characterised in that** it has a light source (21) which, upon generation of an image, illustrates the depicting device (16) in such a way that the staple fibre sample (1) is disposed at the intersection between the light source (21) and the depicting device (16).

30. A device according to claim 29, **characterised in that** the light source (21) is a mirror which is irradiated by a light wave conductor (22) and indirectly illuminates the depicting device (16).

31. A device according to claim 24, **characterised in that** the lighting is carried out by a cold light lamp which, via a mirror, indirectly illuminates the depicting device (16).

32. A device according to claim 22, **characterised in that** the magazine (3) contains the staple fibre samples (1) in a linear arrangement.

33. A device according to claim 22, **characterised in that** the magazine (3) contains the staple fibre samples (1) in a circular arrangement.

34. A device according to claim 22, **characterised in that** a pneumatically or hydraulically driven arm (23) is provided which removes the staple fibre sample (1) from the apparatus (2) after characterisation of the mechanical and/or geometrical properties.

## Revendications

1. Procédé de caractérisation automatique des propriétés mécaniques et/ou géométriques d'échantillons de fibres discontinues, comprenant les étapes suivantes :

a) mise en place des échantillons de fibres discontinues (1) dans un chargeur (3) qui comporte plusieurs organes de serrage (4), dans lesquels est bloquée l'extrémité supérieure de chaque échantillon de fibre discontinue (1), lesquels organes de serrage (4) sont disposés sur une baguette de serrage (5) et peuvent être déplacés sur une distance prédéfinie sur ou avec la baguette de serrage (5),

b) assemblage entre le chargeur (3) rempli et un dispositif (2) de caractérisation des propriétés mécaniques et/ou géométriques des échantillons de fibres discontinues (1), lequel dispositif comporte au moins une pince supérieure (6) et, le cas échéant, une pince inférieure (7) pour bloquer l'échantillon de fibre discontinue (1) à caractériser, parmi lesquelles la pince supérieure (6) est mobile, le chargeur (3) étant assemblé de manière amovible avec le dispositif (2), de telle sorte que les organes de serrage (4) du chargeur (3) se déplacent à proximité de la pince supérieure (6) du dispositif (2),

c) déplacement des organes de serrage (4) du chargeur (3), munis des échantillons de fibres discontinues (1), sur une distance prédéfinie, au moyen d'un dispositif de transport (8),

d) positionnement d'un organe de serrage (4) du chargeur (3) à proximité de la pince supérieure (6) du dispositif (2), au moyen d'un dispositif de réglage (9),

e) déplacement de la pince supérieure (6) du dispositif (2) à partir d'une position de mesure (11) vers une position de transfert (12) au moyen d'un dispositif de transport (10), la pince supérieure (6) étant en position ouverte,

f) fermeture de la pince supérieure (6) du dispositif (2) au moyen d'un dispositif d'ouverture et de fermeture (13), après avoir atteint la position de transfert (12) et avoir saisi l'échantillon de fibre discontinue (1) hors de l'organe de serrage (4) du chargeur (3),

g) déplacement de la pince supérieure (6) du dispositif (2) munie de l'échantillon de fibre discontinue (1), à partir de la position de transfert (12) vers la position de mesure (11), au moyen d'un dispositif de transport (10),

h) mise en oeuvre du processus de caractérisation des propriétés mécaniques et/ou géométriques des échantillons de fibres discontinues (1), au moyen du dispositif (2), et

i) ouverture de la pince supérieure (6) du dispositif (2) à la fin du processus de caractérisation, au moyen du dispositif d'ouverture et de fermeture (13), et évacuation de l'échantillon de fibre discontinue (1) hors du dispositif (2).

2. Procédé selon la revendication 1, **caractérisé en ce que**, pendant la mise en place des échantillons de fibres discontinues (1) dans l'étape a), les extrémités inférieures de ceux-ci sont chargées d'une masse d'une valeur prédéfinie.

3. Procédé de détermination automatique de la frisure des fibres discontinues frisées selon la revendication 1, comprenant les étapes suivantes :

a1) mise en place des échantillons de fibres discontinues (1) frisées dans l'étape a),

d1) présentation de la pince supérieure (6) et de la pince inférieure (7) en position ouverte dans l'étape d),

g1) fermeture de la pince inférieure (7) du dispositif (2) après le déplacement de la pince supérieure (6) vers la position de mesure (11) (étape g)), au moyen d'un dispositif d'ouverture et de fermeture (14), en bloquant l'extrémité inférieure de l'échantillon de fibre discontinue (1) dans la pince inférieure (7),

h1) détermination de la frisure de l'échantillon de fibre discontinue (1) au moyen du dispositif (2) selon une méthode connue en soi dans l'étape h), et

i1) ouverture de la pince supérieure et inférieure (6, 7) du dispositif (2) à la fin du processus de caractérisation, au moyen des dispositifs d'ouverture et de fermeture (13, 14) et évacuation de l'échantillon de fibre discontinue (1) hors du dispositif (2) dans l'étape i).

4. Procédé selon la revendication 3, **caractérisé en ce que** le paramètre de frisure $K_1$ est déterminé selon la relation

$$K_1 = (L_0 - L_1) / L_1$$

dans laquelle $L_0$ désigne la longueur de serrage définie de la fibre frisée et $L_1$ la longueur de la fibre lorsqu'elle atteint la force de défrisage $F_{EK}$, et $F_{EK}$ est la force nécessaire pour défriser la fibre sous l'effet d'une première sollicitation.

5. Procédé selon la revendication 3, **caractérisé en ce que** le paramètre de frisure résiduelle $K_2$ est déterminé selon la relation

$$K_2 = (L_0 - L_2) / L_2$$

dans laquelle $L_0$ désigne la longueur de serrage définie de la fibre frisée et $L_2$ la longueur de la fibre précontrainte lorsqu'elle atteint la force de défrisage $F_{EK}$, et la précontrainte de la fibre pour calculer $K_2$ peut être effectuée dans les conditions suivantes :

- application d'une force de ténacité $F_B$ prédéfinie sur la fibre pendant une durée prédéfinie, et

- suppression de la sollicitation exercée sur la fibre par la mise en place de la pince inférieure (7) sur une longueur de serrage $< L_0$ et phase de restauration de la fibre pendant une période déterminée par le blocage de la pince inférieure (7) dans cette position.

6. Procédé selon la revendication 3, **caractérisé en ce que** le paramètre de résistance à la frisure KB est calculé selon la relation :

$$KB = K_2 / K_1$$

dans laquelle $K_1$ et $K_2$ ont les définitions mentionnées dans les revendications 4 et 5.

7. Procédé selon la revendication 3, **caractérisé en ce que** la mesure de la tension de la fibre, nécessaire pour déterminer la frisure de l'échantillon de fibre discontinue (1), est effectuée au moyen d'un capteur de force (15), sur lequel agissent la pince supérieure (6) et la pince inférieure (7).

8. Procédé de détermination automatique du nombre de courbes de frisure par unité de longueur des fibres discontinues frisées selon la revendication 1, comprenant les étapes suivantes :

   a1) mise en place des échantillons de fibres discontinues (1) frisées dans l'étape a), et
   h2) détermination du nombre de courbes de frisure par unité de longueur de l'échantillon de fibre discontinue (1) dans le dispositif (2) de la manière suivante dans l'étape h),
   h3) réalisation d'une image de longueur prédéfinie et de largeur prédéfinie de l'échantillon de fibre discontinue (1) au moyen d'un dispositif de prise de vues (16), qui se déplace de préférence dans la direction de l'axe longitudinal de la fibre,
   h4) réalisation d'une grille numérique à partir de l'image, dont les pixels sont stockés sous forme de valeurs numériques dans une mémoire (17), les valeurs numériques représentant des chiffres d'indice de la luminosité en chaque lieu de la prise de vue, et
   h5) calcul du nombre de courbes de frisure sur l'échantillon de fibre discontinue représenté sur l'image, à partir de la grille numérique à l'aide d'un traitement numérique de l'image.

9. Procédé selon la revendication 8, **caractérisé en ce que**, lors de la mise en place des échantillons de fibres discontinues (1) dans l'étape a), celles-ci sont chargées chacune d'une masse d'une valeur prédéterminée et en ce que les échantillons de fibres discontinues (1) ainsi chargés sont transférés dans le dispositif (2).

10. Procédé de détermination automatique du nombre de courbes de frisure par unité de longueur des fibres discontinues frisées selon la revendication 8, comprenant les étapes suivantes :

   d1) présentation de la pince supérieure (6) et de la pince inférieure (7) en position ouverte dans l'étape d),
   g1) fermeture de la pince inférieure (7) du dispositif (2) après le déplacement de la pince supérieure (6) vers la position de mesure (11) (étape g)), au moyen d'un dispositif d'ouverture et de fermeture (14), en bloquant l'extrémité inférieure de l'échantillon de fibre discontinue (1) dans la pince inférieure (7), et
   i1) ouverture des pinces supérieure et inférieure (6, 7) du dispositif (2) à la fin du processus de caractérisation, au moyen des dispositifs d'ouverture et de fermeture (13, 14), et évacuation de l'échantillon de fibre discontinue (1) avec un organe de serrage (4) hors du dispositif (2) dans l'étape i).

11. Procédé selon la revendication 8, **caractérisé en ce que**, outre la détermination du nombre de courbes de frisure par unité de longueur de l'échantillon de fibre discontinue (1), on détermine également la tension exercée sur l'échantillon de fibre discontinue (1) lors de la réalisation de l'image, celle-ci étant déterminée au moyen d'un capteur de force (15) sur lequel agissent la pince supérieure (6) et la pince inférieure (7).

12. Procédé selon 1a revendication 8, **caractérisé en ce que** le traitement numérique de l'image dans l'étape h5) est effectué de la manière suivante :

   h6) reproduction sur l'image d'un axe longitudinal de la fibre, qui correspond au tracé que suivrait l'échantillon de fibre discontinue (1) à l'état étiré,
   h7) reproduction d'une ligne médiane sur l'image, laquelle ligne médiane est tracée sur l'axe longitudinal de la fibre selon l'étape h6) ou parallèlement à cet axe longitudinal de la fibre à une distance prédéfinie de celui-ci et qui coupe au moins plusieurs fois l'image de l'échantillon de fibre discontinue (1) générée dans l'étape h3), et
   h8) calcul du nombre de points d'intersection entre la ligne médiane tracée dans l'étape h7) et l'image de l'échantillon de fibre discontinue (1) réalisée dans l'étape h3), en tant que valeur du nombre de courbes de frisure apparaissant sur l'image sur l'échantillon de fibre discontinue (1).

13. Procédé selon la revendication 8, **caractérisé en ce que** le dispositif de prise de vues (16) est une caméra à balayage par lignes.

**14.** Procédé selon la revendication 8, **caractérisé en ce que** le déplacement du dispositif de prise de vues (16) dans le sens de l'axe longitudinal de la fibre est effectué au moyen d'un moteur pas à pas (18).

**15.** Procédé selon la revendication 14, **caractérisé en ce que** le moteur pas à pas (18) agit, par l'intermédiaire d'une broche (19), sur un dispositif de fixation (20), monté contre le dispositif (2) et contre lequel est monté le dispositif de prise de vues (16).

**16.** Procédé selon la revendication 8, **caractérisé en ce que** le dispositif de prise de vues (16) est éclairé pendant la réalisation de l'image par une source de lumière (21), de telle sorte que l'échantillon de fibre discontinue (1) est situé au point d'intersection entre la source de lumière (21) et le dispositif de prise de vues (16).

**17.** Procédé selon la revendication 16, **caractérisé en ce que** la source de lumière (21) utilisée est un miroir, qui est exposé aux rayons d'une fibre optique (22) et qui éclaire indirectement le dispositif de prise de vues (16).

**18.** Procédé selon la revendication 8, **caractérisé en ce que** l'éclairage est fourni par une lampe à lumière froide, qui éclaire indirectement le dispositif de prise de vues (16) par l'intermédiaire d'un miroir.

**19.** Procédé selon la revendication 1, **caractérisé en ce que** le chargeur (3) contient des échantillons de fibres discontinues (1) disposés selon un agencement linéaire.

**20.** Procédé selon la revendication 1, **caractérisé en ce que** le chargeur (3) contient des échantillons de fibres discontinues (1) disposés selon un agencement circulaire.

**21.** Procédé selon la revendication 1, **caractérisé en ce que**, à la fin du processus de caractérisation des propriétés mécaniques et/ou géométriques, l'échantillon de fibre discontinue (1) est retiré hors du dispositif (2) au moyen d'un bras (23) actionné par voie pneumatique ou hydraulique.

**22.** Dispositif de caractérisation automatique des propriétés mécaniques et/ou géométriques d'échantillons de fibres discontinues (1), comprenant les éléments suivants :

A) un chargeur (3) destiné à recevoir des échantillons de fibres discontinues (1), qui comporte plusieurs organes de serrage (4), dans chacun desquels est bloquée l'extrémité supérieure d'un échantillon respectif de fibre discontinue (1), lesquels organes de serrage (4) sont disposés sur une baguette de serrage (5) et lesquels organes de serrage (4) peuvent être déplacés sur une distance prédéfinie sur ou avec la baguette de serrage (5),

B) un dispositif (2) de caractérisation des propriétés mécaniques et/ou géométriques des échantillons de fibres discontinues (1), qui comporte au moins une pince supérieure (6) et, le cas échéant, une pince inférieure (7) par laquelle est bloqué l'échantillon de fibre discontinue (1) à caractériser, parmi lesquelles la pince supérieure (6) est mobile, le chargeur (3) étant assemblé de manière amovible avec le dispositif (2), de telle sorte que les organes de serrage (4) du chargeur (3) se déplacent à proximité de la pince (6) du dispositif (2),

C) un dispositif de transport (8), qui permet de déplacer les organes de serrage (4) du chargeur (3) sur une distance prédéfinie,

D) un dispositif de réglage (9) qui permet de positionner un organe de serrage (4) du chargeur (3) à proximité de la pince supérieure (6) du dispositif (2),

E) un dispositif de transport (10) qui permet de déplacer la pince supérieure (6) du dispositif (2), à partir d'une position de mesure (11) vers une position de transfert (12), et de la déplacer à nouveau en sens inverse, et

F) un dispositif d'ouverture et de fermeture (13) qui permet d'ouvrir et de fermer la pince supérieure (6) du dispositif (2) et de saisir l'échantillon de fibre discontinue (1) hors du chargeur (3) et d'évacuer l'échantillon de fibre discontinue (1) hors du dispositif (2).

**23.** Dispositif selon la revendication 22, **caractérisé en ce que**, pour déterminer la tension exercée sur une fibre, le dispositif (2) comporte un capteur de force (15), sur lequel agit la pince supérieure (6) ou la pince inférieure (7).

**24.** Dispositif selon la revendication 22, **caractérisé en ce que** celui-ci comporte un dispositif de prise de vues (16), qui est destiné à générer une image de longueur prédéfinie et de largeur prédéfinie de l'échantillon de fibre discontinue (1), placé dans le dispositif (2) et qui peut se déplacer dans le sens de l'axe longitudinal de la fibre, et lequel dispositif de prise de vues (16) commande une installation informatique (24) qui génère une grille numérique à partir de l'image, dont les pixels sont stockés sous forme de valeurs numériques dans un dispositif de mémoire (17), les valeurs numériques représentant des chiffres d'indice de la luminosité en chaque lieu de la prise de vue.

**25.** Dispositif selon la revendication 24, **caractérisé en**

**ce que** l'installation informatique (24) permet de calculer le nombre de courbes de frisure sur l'échantillon de fibre discontinue représenté sur l'image, à partir de la grille numérique à l'aide d'un traitement numérique de l'image.

26. Dispositif selon la revendication 24, **caractérisé en ce que** le dispositif de prise de vues (16) est une caméra à balayage par lignes.

27. Dispositif selon la revendication 24, **caractérisé en ce que** celui-ci comporte un moteur pas à pas (18), qui commande le déplacement du dispositif de prise de vues (16) dans la direction de l'axe longitudinal de la fibre.

28. Dispositif selon la revendication 27, **caractérisé en ce que** celui-ci comporte une broche (19), par laquelle le moteur pas à pas (18) agit sur un dispositif de fixation (20) monté sur le dispositif (2) et sur lequel est monté le dispositif de prise de vues (16).

29. Dispositif selon la revendication 24, **caractérisé en ce que** celui-ci comporte une source de lumière (21) qui, pendant la réalisation de l'image, éclaire le dispositif de prise de vues (16), de telle sorte que l'échantillon de fibre discontinue (1) est situé au point d'intersection entre la source de lumière (21) et le dispositif de prise de vues (16).

30. Dispositif selon la revendication 29, **caractérisé en ce que** la source de lumière (21) utilisée est un miroir, qui est exposé aux rayons d'une fibre optique (22) et qui éclaire indirectement le dispositif de prise de vues (16).

31. Dispositif selon la revendication 24, **caractérisé en ce que** l'éclairage est fourni par une lampe à lumière froide, qui éclaire indirectement le dispositif de prise de vues (16) par l'intermédiaire d'un miroir.

32. Dispositif selon la revendication 22, **caractérisé en ce que** le chargeur (3) contient des échantillons de fibres discontinues (1) disposés selon un agencement linéaire.

33. Dispositif selon la revendication 22, **caractérisé en ce que** le chargeur (3) contient des échantillons de fibres discontinues (1) disposés selon un agencement circulaire.

34. Dispositif selon la revendication 22, **caractérisé en ce qu'**il est prévu un bras (23) actionné par voie pneumatique ou hydraulique, par lequel l'échantillon de fibre discontinue (1) est retiré hors du dispositif (2) à la fin du processus de caractérisation des propriétés mécaniques et/ou géométriques.